# EUROPEAN PATENT SPECIFICATION

(11) **EP 1 721 015 B1**
(45) Date of publication and mention of the grant of the patent: **15.09.2010**
(21) Application number: 05712046.1
(22) Date of filing: 21.01.2005
(51) Int. Cl.: C12Q 1/70

(54) **PRIMER AND PROBE DESIGN FOR EFFICIENT AMPLIFICATION AND DETECTION OF HCV 3' NON-TRANSLATING REGION**
PRIMER- UND SONDENKONSTRUKTION ZUR EFFIZIENTEN AMPLIFIKATION UND DETEKTION DES 3'-NICHTTRANSLATIERTEN BEREICHS VON HCV
AMORCES ET SONDES CONFIGUREES POUR PERMETTRE UNE AMPLIFICATION ET UNE DETECTION EFFICACES DE LA REGION NON TRADUISANTE 3' DU VHC

(30) Priority: 23.01.2004 US 538816 P; 23.01.2004 US 538815 P; 23.01.2004 US 538814 P
(43) Date of publication of application: 15.11.2006
(73) Proprietor: bioMerieux, Inc., Durham, NC 27712 (US)
(72) Inventor: WANG, Hwa-Tang, Thomas, Encinitas, CA 92924 (US); WASHBURN, Brian, Cambridge, MA 02140 (US)
(74) Representative: Froud, Clive
(86) International application number: PCT/US2005/002415
(87) International publication number: WO 2005/071117

(56) References cited:
- EP-A- 1 026 262
- WO-A-00/00638
- WO-A-01/64959
- WO-A-03/100014
- US-A- 5 837 463
- US-A1- 2003 152 912
- US-B1- 6 297 003
- DATABASE Geneseq [Online] 1 January 2004 (2004-01-01), "Anti-HCV agent LZ-PAIR-95 DNA-RNA hybrid." XP002342610 retrieved from EBI accession no. GSN:ADD00767 Database accession no. ADD00767 & WO 03/016572 A (ELI LILLY AND COMPANY; ZHAO, GENSHI; LU, JIN; GLASS, JOHN, IRVIN; MART) 27 February 2003 (2003-02-27)
- DATABASE Geneseq [Online] 2 April 2001 (2001-04-02), "PCR primer for infectious Hepatitis C virus strain HC-J6CH." XP002342611 retrieved from EBI accession no. GSN:AAC86676 Database accession no. AAC86676 & WO 00/75338 A (THE GOVERNMENT OF THE UNITED STATES OF AMERICA AS REPRESENTED BY THE S) 14 December 2000 (2000-12-14)
- DATABASE Geneseq [Online] 23 September 2003 (2003-09-23), "Anti-HCV nucleic acid molecule #130." XP002342612 retrieved from EBI accession no. GSN:ACD66172 Database accession no. ACD66172 & WO 02/081494 A (RIBOZYME PHARMACEUTICALS, INC; BLATT, LAWRENCE; MACEJAK, DENNIS; MCSWI) 17 October 2002 (2002-10-17)
- DATABASE Geneseq [Online] 28 March 2000 (2000-03-28), "HCV 3' non core region substrate for Hammerhead ribozyme HCV.3-161." XP002342613 retrieved from EBI accession no. GSN:AAZ61860 Database accession no. AAZ61860 & WO 99/55847 A (RIBOZYME PHARMACEUTICALS, INC; BLATT, LAWRENCE; MCSWIGGEN, JAMES, A; R) 4 November 1999 (1999-11-04)
- DATABASE Geneseq [Online] 5 September 2001 (2001-09-05), "T7 promoter sequence." XP002342614 retrieved from EBI accession no. GSN:AAH44987 Database accession no. AAH44987 & WO 01/36442 A (JI, JIUPING; MANAK, MARK; WU, KEZUO; CHEN, XIULI; YANG, LIJUAN; GONZAL) 25 May 2001 (2001-05-25)
- DATABASE Geneseq [Online] 23 September 2003 (2003-09-23), "Anti-HCV nucleic acid molecule target sequence #87." XP002342812 retrieved from EBI accession no. GSN:ACD66064 Database accession no. ACD66064 & WO 02/081494 A (RIBOZYME PHARMACEUTICALS, INC; BLATT, LAWRENCE; MACEJAK, DENNIS; MCSWI) 17 October 2002 (2002-10-17)
- KOLYKHALOV A A ET AL: "IDENTIFICATION OF A HIGHLY CONSERVED SEQUENCE ELEMENT AT THE 3' TERMINUS OF HEPATITIS C VIRUS GENOME RNA" JOURNAL OF VIROLOGY, THE AMERICAN SOCIETY FOR MICROBIOLOGY, US, vol. 70, no. 6, June 1996 (1996-06), pages 3363-3371, XP000985963 ISSN: 0022-538X cited in the application

## Description

### FIELD OF THE INVENTION

The present invention relates to oligonucleotides that bind to the 3' non-translating region (3'NTR) of Hepatitis C Virus (HCV), useful for the detection and/or quantification of HCV. Also provided are methods of nucleic acid assays useful in the detection, capture and amplification of HCV utilizing the 3'NTR as the target region.

### BACKGROUND OF THE INVENTION

After the development of diagnostic tests for hepatitis A and hepatitis B viruses in the 1970s, an additional parenterally transmitted agent responsible for the majority of transfusion-associated non-A, non-B hepatitis cases was recognized. The identification of this agent turned out to be very difficult (Bartenschlager and Lohmann, J. Gen Virol. 81: 1631-1648 (2000). With the advent of recombinant DNA, the genome of the virus that was termed hepatitis C virus (HCV) was cloned (Choo et al., Science 244: 359-362 (1989)). Its RNA has been difficult to study because biological materials are scarce and RNA replication is of low efficiency (Shi and Lai, Cell. Mol. Life Sci. 58: 1276-1295 (2001)).

Many general techniques are known for studying nucleic acids. When necessary, enzymatic amplification of nucleic acid sequences will enhance the ability to detect a desired nucleic acid sequence. Generally, the currently known amplification schemes can be broadly grouped into two classes based on whether the enzymatic amplification reactions are driven by continuous cycling of the temperature between the denaturation temperature, the primer annealing temperature, and the amplicon (product of enzymatic amplification of nucleic acid) synthesis temperature, or whether the temperature is kept constant throughout the enzymatic amplification process (isothermal amplification). Typical cycling nucleic acid amplification technologies (thermocycling) are polymerase chain reaction (PCR), and ligase chain reaction (LCR). Specific protocols for such reactions are discussed in, for example, Short Protocols in Molecular Biology, 2.sup.nd Edition, A Compendium of Methods from Current Protocols in Molecular Biology, (Eds. Ausubel et al., John Wiley & Sons, New York, 1992) chapter 15. Reactions which are isothermal include: transcription-mediated amplification (TMA), nucleic acid sequence-based amplification (NASBA), and strand displacement amplification (SDA).

U.S. Patent Nos 4,683,195 (Mullis); 4,965,188 (Mullis); and 4,683,202 (Mullis) describe a polymerase chain reaction (PCR) utilizes DNA polymerase, complementary primer molecules and repeated cycles of thermal reactions to exponentially replicate target nucleic acid molecules. Isothermal target amplification methods include transcription-based amplification methods, in which an RNA polymerase promoter sequence is incorporated into primer extension products at an early stage of the amplification (WO 89/01050), and further target sequence, or target complementary sequence, is amplified by transcription steps and digestion of an RNA strand in a DNA/RNA hybrid intermediate product. See, for example, U.S. Pat. Nos. 5,169,766 and 4,786,600. These methods include transcription mediated amplification (TMA), self-sustained sequence replication (3SR), Nucleic Acid Sequence Based Amplification (NASBA), and variations there of. See, for example, Guatelli et al. Proc. Natl. Acad. Sci. U.S.A. 87:1874-1878 (1990); U.S. Pat. Nos. 5,766,849 5,399,491; 5,480,784; 5,766,849; 5,466,586 (NASBA); 5,409,818(NASBA); 5,554,517(NASBA); 6,063,603(NASBA); 5,130,238 (NASBA); and 5,654,142 (TMA); and 5,130,238 (Malek et al.); 5,409,818 (Davey et al.); 5,654,142 (Kievits); and 6,312,928 (Van Gemen et al.) (nucleic acid sequence-based amplification (NASBA) techniques). U.S. Patent 5,792,607 (Backman) describes amplification methods referred to as ligase chain reactions (LCR). U.S. Patent Nos 5,744,311 (Fraiser); 5,648,211 (Fraiser) and 5,631,147 (Lohman), describe isothermal amplification systems based on strand displacement amplification (SDA). Other approaches include Q.beta. replicase, strand displacement assay (SDA), transcription mediated iso CR cycling probe technology, nucleic acid sequence-based amplification (NASBA) and cascade rolling circle amplification (CRCA). Additional U.S. Patent documents which describe nucleic acid amplification include U.S. Pat. Nos. 4,876,187; 5,030,557; 5,399,491; 5,485,184; 5,554,517; 5,437,990; 5,399,491 and 5,554,516.

Nucleic acid hybridization techniques have been described for example, in Sambrook et al. Molecular Cloning A Laboratory Manual, 2nd Ed. Cold Spring Lab. Press, Dec. 1989; U.S. Patent Nos 4,563,419 (Ranki) and 4,851,330 (Kohne) and in Dunn, et al., Cell 12, pp. 23-26 (1978) among many other publications.

Detection methods utilizing nucleic acids are also known. Nucleic acids are often labeled for various detection purposes. For example, methods described in U.S. Patent Nos 4,486,539 (Kourlisky); 4,411,955 (Ward); 4,882,269 (Schneider) and 4,213,893 (Carrico), illustrate preparation of labeled detection probes for detecting specific nucleic acid sequences. Furthermore, before or after exposing an extracted nucleic acid to a probe, the target nucleic acid can be immobilized by target-capture means, either directly or indirectly, using a "capture probe" bound to a substrate, such as a magnetic bead. Examples of target-capture methodologies are described by Ranki et al., U.S. Pat. No. 4,486,539, and Stabinsky, U.S. Pat. No. 4,751,177. Further uses of probes have been described, for example, in U.S. Patent Nos. 5,210,015; 5,487,972; 5,804,375; 5,994,076.

HCV has been classified as the sole member of a distinct genus called *Hepacivirus* in the family *Flaviviridae* HCV is an enveloped particle harboring a plus-strand RNA with a length of approximately 9600 nucleotides. The genome carries a single long open reading frame (ORF) encoding a polyprotein that is proteolytically cleaved into a set of distinct products. An approximately 340 nucleotide-long 5' non-translated region (NTR) functions as an internal ribosome entry site (IRES) for translation of the HCV ORF (Tsukiyama-Kohara et al., J. Virol. 66: 1476-1483 (1992); Wang et al., J. Virol. 67: 3338-3344 (1993)). The 3'NTR was more recently studied (Kolykhalov et al., J. Virol. 70: 3363-3371 (1996); Tanaka et al., Biochem. Biophys. Res. Comm. 215: 744-749 (1995), J. Virol. 70: 3307-3312 (1996); Yamada et al., Virology 223: 255-261 (1996)). The HCV 3' nontranslated region has been found to include four elements (positive sense, 5' to 3'): (i) a short sequence with significant variability among genotypes, (ii) a homopolymeric poly(U) tract, (iii) a polypyrimidine stretch consisting ofmainly U with interspersed C residues, (iv) a novel sequence of 98 bases (the X-region). This latter nucleotide sequence is not present in human genomic DNA and is highly conserved among HCV genotypes (Kolykhalov *et al.* (1996); Pavio and Lai, J.Biosci.28(3): 287-304 (2003)). Most HCV infections persist, leading in about 50% of all cases to chronic hepatitis, which can develop into chronic active hepatitis, liver cirrhosis and hepatocellular carcinoma. Furthermore, HCV is distributed worldwide, with the number of infected individuals being estimated to be ∼170 million. (Bartenschlager and Lohmann (2000). Thus, a definitive diagnostic test is needed to identify infected individuals. Furthermore, HCV genotyping in patients is essential for diagnostic and epidemiological studies, as well as in studies of the natural history and treatment of HCV.

Detection of HCV RNA is the diagnostic test utilized for acute and chronic HCV infection. However, it is often complicated by the low levels of HCV replication or small numbers of infected cells in hepatitis C patients (Shi and Lai (2000)). Most qualitative and quantitative diagnostic tests are RTPCR aiming at the 5'-UTR, the most conserved region of the HCV genome. However, the sensitivity of this method has been reported to be accompanied by problems of false priming, presumably in areas of RNA secondary structure (Shi and Lai (2000)). This has been partially addressed by the use of tagged primers or a thermostable reverse transcriptase (Lanford, Virology 202: 606-614 (1994)). Since the core and NS5B genes are relatively conserved and do not contain extensive secondary structures, they have also been employed in various detection methods.

The X region of the 3'-UTR is another highly conserved region in the HCV genome, but it is highly structured. The 3' HCV NTR is considered a difficult amplification target for at least two reasons: it forms highly stable secondary structures; and the NTR is very small. It has not been used in the detection of HCV RNA due, at least in part, to its lack of practical advantages over the well-established tests based on the 5'-UTR sequence (Shi and Lai (2000)).

Thus there is a clear need for primer and probe designs that provide more useful and reliable results in detection of the presence of HCV. Specifically, there is a need for primers and probes that are aimed at overcoming the complications of 3' NTR secondary structure, type specificity and palindrome sequences that could affect the hybridization process and providing improved means for the detection, capture, and/or amplification of HCV nucleic acids. The present invention provides oligonucleotides that address these needs to help solve such issues.

[0011a] Reference may also be made to the following: EP-A-1 026 262, which relates to oligonucleotide primers for efficient detection of hepatitis C virus (HCV) and methods of use thereof; US-B1-6, 297, 003, which relates to methods for the detection of a novel hepatitis C virus (HCV) terminal 3' non-coding region; US-A-5,837,463, which relates to nucleic acid of C type hepatitis virus derivation and process for detection virus using said nucleic acid; WO-A-03/100014, which relates to method for quantitating negative strand RNA synthesis; US-A1-2003/0152912, which relates to multiple viral replicon culture systems; WO-A-00/00638, which relates to tagging of RNA amplicons generated by transcription-based amplification; WO-A-01/64959, which relates to detection of hepatitis B virus RNA; DATABASE Geneseq [Online] 1 January 2004 (2004-01-01), "Anti-HCV agent LZ-PAIR-95 DNA-RNA hybrid." XP002342610 retrieved from EBI accession no. GSN:ADD00767 Database accession no. ADD00767; DATABASE Geneseq [Online] 2 April 2001 (2001-04-02), "PCR primer for infectious Hepatitis C virus strain HC-J6CH." XP002342611 retrieved from EBI accession no. GSN:AAC86676 Database accession no. AAC86676; DATABASE Geneseq [Online] 23 September 2003 (2003-09-23), "Anti-HCV nucleic acid molecule #130." XP002342612 retrieved from EBI accession no. GSN:ACD66172 Database accession no. ACD66172; DATABASE Geneseq [Online] 28 March 2000 (2000-03-28), "HCV 3' non core region substrate for Hammerhead ribozyme HCV.3-161."XP002342613 retrieved from EBI accession no. GSN:AAZ61860 Database accession no. AAZ61860; and DATABASE Geneseq [Online] 5 September 2001 (2001-09-05), "T7 promoter sequence. "XP002342614 retrieved from EBI accession no. GSN:AAH44987 Database accession no. AAH44987.

### SUMMARY OF THE INVENTION

The present invention provides an isolated Hepatitis C virus-derived nucleic acid comprising a nucleotide sequence selected from the group consisting of specific nucleic acid sequences corresponding to a portion of the 3'NTR of HCV. Such oligonucleotides are useful in detecting the presence of HCV nucleic acids. Specifically, the present invention provides an isolated nucleic acid consisting of a nucleotide sequence selected from the group consisting of SEQ ID NO: 11, SEQ ID NO: 27, SEQ ID NO: 29, SEQ ID NO: 30, SEQ ID NO: 31, SEQ ID NO:32.

Further disclosed herein is an isolated nucleic acid comprising (a) a tag oligonucleotide having a 5' end and a 3' end, and (b) a primer nucleic acid having a 5' end and a 3' end and comprising a nucleotide sequence corresponding to a portion of the 3'NTR of HCV, wherein the tag oligonucleotide consists of about 18 to about 23 randomly selected nucleotides heterologous to HCV and is linked at its 3' end to the 5' end of the primer nucleic acid. More specifically, disclosed is an isolated nucleic acid comprising (a) a tag oligonucleotide having a 5' end and a 3' end, and (b) a primer nucleic acid having a 5' end and a 3' end and comprising a nucleotide sequence selected from the group consisting of SEQ ID NO:1, SEQ ID NO:2, SEQ ID NO:3, SEQ ID NO:4, SEQ ID NO:5, SEQ ID NO:6, SEQ ID NO:7, and SEQ ID NO: 8, wherein the tag oligonucleotide consists of about 18 to about 23 randomly selected nucleotides heterologous to HCV and is linked at its 3' end to the 5' end of the primer nucleic acid.

The present invention additionally provides a method of assaying for the presence of HCV in a nucleic acid sample comprising (a) amplifying a selected HCV nucleic acid to form an HCV amplification product, utilizing as a primer of amplification a nucleic acid sequence of the present invention corresponding to a portion of the 3'NTR of HCV, and (b) detecting the presence of amplified product. Specifically, the present invention provides a method of assaying for the presence of HCV in a nucleic acid sample comprising (a) amplifying a selected HCV nucleic acid to form an HCV amplification product, utilizing as a primer of amplification a nucleic acid consisting of a nucleotide sequence selected from the group consisting of SEQ ID NO: 11, SEQ ID NO: 27, SEQ ID NO: 29, SEQ ID NO: 30, SEQ ID NO: 31, SEQ ID NO:32, and (b) detecting the presence of amplified product.
The instant invention further provides a method of quantifying the amount of HCV nucleic acid in a sample comprising (a) amplifying selected HCV nucleic acid to form an HCV amplification product, utilizing as a primer of amplification a nucleic acid sequence of the present invention corresponding to a portion of the 3'NTR of HCV, and (b) quantifying the amount of amplified product.
The instant invention specifically provides a method of quantifying the amount of HCV nucleic acid in a sample comprising (a) amplifying selected HCV nucleic acid to form an HCV amplification product, utilizing as a primer of amplification a nucleic acid consisting of a nucleotide sequence selected from the group consisting of SEQ ID NO: 11, SEQ ID NO: 27, SEQ ID NO: 29, SEQ ID NO: 30, SEQ ID NO: 31, SEQ ID NO:32, and (b) quantifying the amount of amplified product.

Additionally disclosed is a method of assaying for the presence of HCV in a nucleic acid sample comprising (a) amplifying a target nucleic acid comprising a selected portion of the 3'NTR of HCV and (b) detecting the presence of amplified product utilizing a probe comprising a nucleotide sequence selected from the group consisting of SEQ ID NO: 21, SEQ ID NO: 22; SEQ ID NO:19, SEQ ID NO: 24, SEQ ID NO: 41, SEQ ID NO: 43, SEQ ID NO: 44.

### BRIEF DESCRIPTION OF THE DRAWINGS

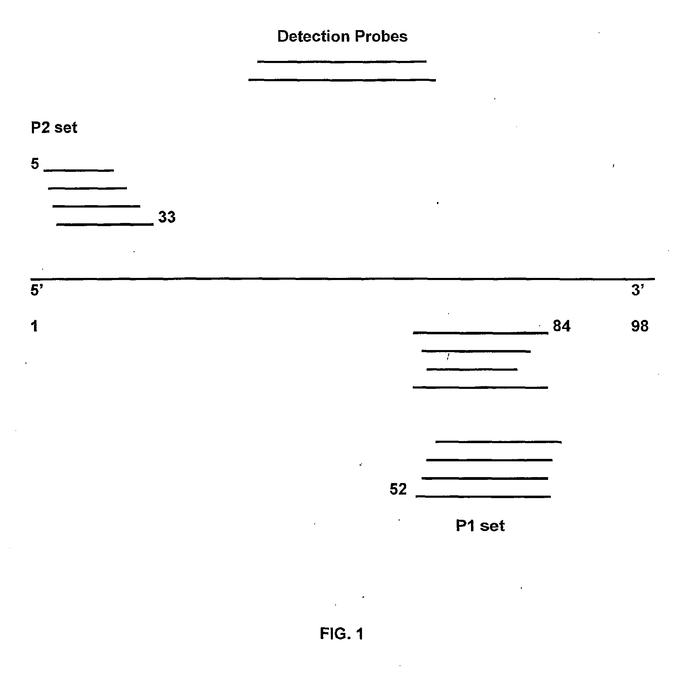

Fig. 1 provides a schematic of the 3' NTR of HCV (98 sequences), showing the general region of hybridization of P1 primers, P2 primers and detection probes.

### DETAILED DESCRIPTION OF THE INVENTION

The 3' HCV NTR is considered a difficult amplification target for at least two reasons. First, it forms highly stable secondary structures. The intramolecular pairing (including potential double-stranded (stem) and palindrome regions) is expected to cause difficulties for primer binding, primer extension, and probe binding. Secondly, the NTR is very small (98 nt), resulting in unusually limited primer and probe choices, and an amplicon of sub-optimal length (amplicons are ideally >100 nt). The inability to reduce secondary structure by raising the temperature (as in PCR) makes it particularly problematic for isothermal amplification methods such as TMA and NASBA. The present invention provides primers and probes that help overcome these challenges.
The 3' NTR of Hepatitis C virus has the following sequence:
**Genotype 1:** 
GGUGGCUCCAUCUUAGCCCUAGUCACGGCUAGCUGUGAAAGGUCCGUGAGCCGCAUGACUG
CAGAGAGUGCUGAUACUGGCCUCUCUGCAGAUCAUGU (SEQ ID NO: 25)
**Genotype 2:**

GGUGGCUCCAUCUUAGCCCUAGUCACGGCUAGCUGUGAAAGGUCCGUGAGCCGCAUGACUG
CAGAGAGUGCCGUAACUGGUCUCUCUGCAGAUCAUGU (SEQ ID NO: 26)

The most common sequence differences between Genotype 1 and Genotype 2 are indicated by underlining. Although the region is highly conserved, additional nucleotide differences may also be present in the individual isolates.

As stated above, the present invention provides oligonucleotides, derived from the 3' NTR of Hepatitis C virus, found to be particularly useful in assays to detect the presence, or quantify the amount of, HCV nucleic acids in selected samples.

Throughout this application, nucleic acid sequences may have descriptors that include an "nt" range of numbers. Such descriptor indicates where, within the 98 nucleotides of the 3'HCV NTR, the sequence corresponds, the first of the 98 nucleotides, reading in a 5'-3' direction along the viral genome, having the number "1". For example, the following oligonucleotide "1206 (nt5-24)" indicates that the sequence of the oligonucleotide named 1206 has a nucleic acid sequence that corresponds to nucleotides 5-24 of the 3'NTR. Additionally, it is noted that references to sequences that include thymidine can be readily adapted to utilize uridine in substitution for thymidine, where useful for the particular assay. Furthermore, nucleotides may be modified by addition of chemical groups, or substitution of individual residues by analogues (e.g., 2'-O-methoxy versions). Additional such modified nucleotides are known in the art; some examples include hydroxymethyl nucleotides, methylated nucleotides, fluorinated nucleotides, alpha this phosphate nucleotides, amine-modified nucleotides, methoxy nucleotides, carboxymethyl nucleotides, thio nucleotides, inosine, dihydrouridine, psuedouridine, wybutosine, queuosine, C7dGTP. Additional modified nucleotides are found in U.S. Pat. Nos 5,405,950 and 5,633,364 (both, Mock and Lovern).

The invention particularly provides an isolated Hepatitis C virus-derived nucleic acid consisting of specific nucleic acid sequences corresponding to a portion of the 3'NTR of HCV. Specifically, the present invention provides an isolated nucleic acid consisting of a nucleotide sequence selected from the group consisting of SEQ ID NO: 11, SEQ ID NO: 27, SEQ ID NO: 29, SEQ ID NO: 30, SEQ ID NO: 31, and SEQ ID NO:32. NASBA conditions are known in the art and can include, for example, parameters creating stringency provided by about 41°C and about 70 mM KCl (see. e.g., U.S. Pat. Nos.5,466,586; 5,409,818; 5,554,517; 6,063,603; and 5,130,238; Deiman B, van Aarle P, Sillekens P. Characteristics and applications of nucleic acid sequence-based amplification (NASBA). Mol Biotechnol. 2002 Feb;20(2):163-79.).

Oligonucleotides of the present invention can be utilized, for example, as primers and/or as probes for the detection of HCV nucleic acids in a sample. Throughout this application, a particular oligonucleotide may be exemplified in use as a particular type (e.g. P1-type (linked to a sequence that provides a promoter region when in double-stranded form) or P2 type (used alone or linked to a tag oligonucleotide)) primer or as a probe; however, such use should not limit the use(s) for which the oligonucleotide may be useful. For example, a primer exemplified as a P1 primer may be useful as a P2-type primer. Additionally, an oligonucleotide exemplified for use as a probe may be useful as the base HCV-hybridizing sequence for other styles of probes (e.g., having different labels or capture oligonucleotides or other structures for functioning of that probe type).

The present invention provides amplicon length modulation through the use of the attachment of non-related sequence at the 5' end of primers. Such non-related sequences are particularly useful for non-promoter-linked primers, such as P2 primers herein. With this invention, our results demonstrate that one can, given the teachings herein, modulate amplicon length and modulate its secondary structure for highly efficient probe-based detection.

Thus, further disclosed herein is an isolated nucleic acid comprising (a) a tag oligonucleotide having a 5' end and a 3' end, and (b) a primer nucleic acid having a 5' end and a 3' end and comprising a nucleotide sequence corresponding to a portion of the 3'NTR of HCV, wherein the tag oligonucleotide consists of about 1 to about 23 randomly selected nucleotides heterologous to HCV and is linked at its 3' end to the 5' end of the primer nucleic acid. More specifically, the present invention further provides an isolated nucleic acid comprising (a) a tag oligonucleotide having a 5' end and a 3' end, and (b) a primer nucleic acid having a 5' end and a 3' end and comprising a nucleotide sequence selected from the group consisting of SEQ ID NO:1, SEQ ID NO:2, SEQ ID NO:3, SEQ ID NO:4, SEQ ID NO:5, SEQ ID NO:6, SEQ ID NO:7, and SEQ ID NO: 8, wherein the tag oligonucleotide consists of about 18 to about 23 randomly selected nucleotides heterologous to HCV and is linked at its 3' end to the 5' end of the primer nucleic acid.

Preferably the tag oligonucleotide (or "tail") is a sequence heterologous to the target nucleic acid, and, more preferably, heterologous to any region of HCV. The tag oligonucleotide can comprise any selected heterologous sequence of about 18-23 nucleotides. An approximately 20-mer oligonucleotide "tail" can readily be designed by one skilled in the art, given these teachings, to create additional detection tails useful with (non-promoter-carrying) primers to increase detectable amplicons from an amplification reaction. The tail is preferably comprised of sequences heterologous to the target nucleic acid. The oligonucleotide tail can be from about 18 to about 23 nucleotides in length, is preferably about 19-22 nucleotide in length, more preferably about 20-21 nucleotides in length, and most preferably 20 nucleotides in length. One example of an oligonucleotide tail is the "ECL (electrochemiluminescence) tail," set forth in SEQ ID NO:9.

Preferably, the tag comprises an ECL tag; more particularly, the nucleotide sequence set forth in SEQ ID NO: 9. Preferably the oligonucleotide comprises a tag oligonucleotide linked at its 3' end to the 5' end of a primer having a nucleic acid sequence set forth in SEQ ID NO: 8. Thus, disclosed herein is a preferred isolated nucleic acid comprising a nucleotide sequence set forth in SEQ ID NO: 37, which consists of an ECL tag linked at its 3' end to the 5' end of the primer (herein referred to as "1259") having a nucleotide sequence set forth in SEQ ID NO:8.

The present invention further provides an isolated nucleic acid comprising (a) a promoter oligonucleotide that, when in double-stranded form, can function as a T7 promoter, and (b) a primer nucleic acid having a 5' end and a 3' end and consisting of a nucleotide sequence corresponding to a portion of the 3'NTR of HCV, which is selected from the group consisting of SEQ ID NO:11, SEQ ID NO:27, SEQ ID NO:29, SEQ ID NO:30, SEQ ID NO:31, and SEQ ID NO:32, wherein the promoter oligonucleotide is linked at its 3' end to the 5' end of the primer nucleic acid.

The nucleic acid sequence of the T7 promoter is well-known to persons skilled in the art, and though a particular sequence is exemplified herein, functional equivalents having slight variations may be designed. In a preferred embodiment, the sequence of the T7 promoter is that set forth in SEQ ID NO:38. A further example is provided by SEQ ID NO: 23. An oligonucleotide having T7 promoter sequences is useful as a primer, and, when utilized as such, is herein referred to as a "P1 primer," P1-type primer," "promoter-oligonucleotide," or simply as "P1." In a transcription-based amplification reaction (e.g., NASBA, TMA), as is known in the art, these T7 promoter sequences have a function in the amplification reaction, priming the transcription of RNA from the target template, in this case, HCV. The nucleotide sequences of primers exemplified herein as of the "P1" type are typically listed without the T7 promoter sequences, but in the experiments, such T7 promoter sequences are present.

Additionally disclosed herein is a method of assaying for the presence of HCV in a nucleic acid sample comprising (a) amplifying a target nucleic acid comprising a selected portion of the 3'NTR of HCV and (b) detecting the presence of amplified product utilizing a probe comprising a nucleotide sequence selected from the group consisting of SEQ ID NO: 21, SEQ ID NO: 22; SEQ ID NO:19, SEQ ID NO: 24, SEQ ID NO: 41, SEQ ID NO: 43, SEQ ID NO: 44. In such a method, any useful HCV primer(s) can be utilized; examples are provided herein. Assay conditions are known to those of skill in the are and exemplified herein.

Oligonucleotides of the present invention can have various uses. They can, for example, be utilized as probes in capture and detection reactions, and as primers and/or probes in various amplification reactions. Provided herein are primers and probes utilized for 3' HCV amplification and exemplified by TMA-and NASBA. Both of these methods use isothermal amplification to produce RNA amplicons through the combined use of reverse transcriptase and T7 polymerase. Because of these similarities, the primers and probes are expected to share some performance similarity in the two systems, and experiments (see Examples) support this.

The present invention additionally provides a method of assaying for the presence of HCV in a nucleic acid sample comprising (a) amplifying a selected HCV nucleic acid to form an HCV amplification product, utilizing as a primer of amplification a nucleic acid consisting of a nucleotide sequence corresponding to a portion of the 3'NTR of HCV, is selected from the group consisting of SEQ ID NO: 11, SEQ ID NO:27, SEQ ID NO:29, SEQ ID NO:30, SEQ ID NO:31, and SEQ ID NO:32, and (b) detecting the presence of amplified product.

Nucleic acid hybridization techniques and conditions are known to the skilled artisan and have been described for example, in Sambrook et al. Molecular Cloning A Laboratory Manual, 2nd Ed. Cold Spring Lab. Press, Dec. 1989; U.S. Patent Nos 4,563,419 (Ranki) and 4,851,330 (Kohne) and in Dunn, et al., Cell 12, pp. 23-26 (1978) among many other publications. Various modifications to the hybridization reactions are known in the art including in- solution hybridization or hybridization to capture probes on a solid support in one or more reaction steps.

Detection methods utilizing nucleic acids are also known. Nucleic acids can be labeled for use in detection. For example, detectable labels have been conjugated, directly or indirectly through linker arms on either the base, sugar or phosphate moiety of one or more specific oligonucleotides (see, e.g., U.S. Patent Nos 4,486,539 (Kourlisky); 4,411,955 (Ward); 4,882,269 (Schneider) and 4,213,893 (Carrico)). Labels known in the art include fluorochromes, radioisotopes, dyes, enzymes such as alkaline phosphatase, and luminescent or chemiluminescent molecules. Detectably labeled probes may, for example, be used to bind to amplified nucleic acid reaction products or amplicons during or after an amplification reaction. Particularly useful probes are those that can detect the target under the preferred conditions for the assay and type of probe utilized; typical hybridization conditions can be stringent hybridization conditions such as taught by Sambrook, et al.

This invention involves the primer and probe designs for efficient capture, amplification and detection of HCV 3' end 98-base non-translated region. The primer designs can be applied for TMA, NASBA, SDA, PCR and other amplification methods. The present oligonucleotides are useful in the present detection and quantification assays. Amplification can be performed by thermocycling methods or isothermal methods. The present oligonucleotides are particularly useful, and preferably used in, transcription based amplification methods, for example, NASBA and TMA. Transcription based amplification methods often utilize single stranded RNA as the input material, although single or double stranded DNA can likewise be used as input material. When a transcription based amplification method is practiced on a sample with single stranded RNA (of the "plus" sense) with additional sequences on both the 3'-end and the 5' end of the target sequence, a pair of oligonucleotides that is conveniently used with the methods can include (1) a first oligonucleotide (often referred to as a "promoter-oligonucleotide", or "P1" primer) that is capable of hybridizing to the 3-end of the target sequence, which oligonucleotide has the sequence of a promoter (preferably the T7 promoter) attached to its 5' end (the hybridizing part of this oligonucleotide has the opposite polarity as the plus RNA used as input material); and (2) a second oligonucleotide ("primer") which comprises the 3' end of the target sequence (this oligonucleotide has the same polarity as the plus RNA).

When such a pair of oligonucleotides, together with all enzymes having the appropriate activities, and a sufficient supply of the necessary ribonucleotides and deoxyribonucleotides are put together in one reaction mixture and are kept under the appropriate conditions (that is, under the appropriate buffer conditions and at the appropriate temperature) (such conditions being known in the art) for a sufficient period of time an isothermal continuous amplification reaction will start.

For use in non-transcription-based methods, the first primer can be an oligonucleotide provided here and can lack a T7 promoter sequence. For any amplification method, the present olgionucleotides can provide probes for detection of amplicons. The probe designs provided herein are for target capture (sample preparation), amplicon capture, and amplicon detection, which methods are know in the art. Probe designs for different detection methods: HPA, TAQman, molecular Beacons and Sandwich hybridization have also been discovered. These designs demonstrate the efficient capture and detection by hybridization to amplicons

### EXAMPLES

Within this application and particularly within the Examples, the primers utilized may be referred to as P1 primers or P2 primers. This terminology simply indicates that, within a NASBA or TMA reaction in these examples, the P1 primer is the primer having a T7 promoter sequence attached to its 5' end (promoter primer); the P2 primer does not. It is not limiting terminology.

### TMA amplification and detection of 3' HCV

To evaluate the usefulness of various primers in HCV amplification, standard VIDAS Probe D2 qHCV assay conditions were used as follows. For lysis, 0.5 ml sample (such as EDTA-plasma, or in vitro RNA transcript diluted in base matrix) was mixed with 0.4 ml urea-based lysis buffer, incubated 67.5°C (20 min) and cooled to room temp (20 min). During these incubations the released nucleic acid was captured by capB probe (SEQ ID NO: 19), which was linked to magnetic beads by a polyA tail. Complexes were washed and resuspended in RAR buffer (see Table 1). RNA target was then denatured at 65°C , then amplified by TMA (standard conditions; McDonough et al., Nucleic acid amplification technologies 1998:113-123 BioTechniques Books Natick, MA, Lee H Morse S Olsvik ∅ eds.) at 42°C with primers 1259 (SEQ ID NO: 8) and 1236 (SEQ ID NO: 11). Specifically, the RAR-resuspended purified target was then added to VIVAS Probe strips (containing all additional reagents required for amplification and detection, such as enzymes, probes, wash solutions, and detection substrate). The amplification was carried out in bioMerieux AmpStations, and then transferred to VIDAS instruments, which carried out sequence-specific capture of the amplicons, washing, and detection with a fluorescent-conjugate probe specific for the 3' end. The SPR Probe 1247 (SEQ ID NO: 22) captures the amplicon on the VIDAS SPR. The AKP Probe (1246) (SEQ ID NO: 21) was conjugated to alkaline phosphatase and binds to the purified amplicon, providing detection via fluorescent AKP substrate.

**Table 1: RAR Buffer**

| | |
|---|---|
| **Tween 80** | **12.5%** |
| **Tris pH 7.9** | **95 mM** |
| **EDTA** | **0.375 MM** |
| **KCl** | **50 MM** |
| **ZnAc** | **0.0765 MM** |
| **MgCl** | **20.75 MM** |
| **Glycerol** | **10%** |
| **DMSO** | **5%** |
| **ATP** | **4MM** |
| **CTP** | **3MM** |
| **GTP** | **6MM** |
| **UTP** | **2MM** |
| **d-ATPs** | **1MM** |
| **d-CTPs** | **1MM** |
| **d-GTPs** | **1MM** |
| **d-TTPs** | **1MM** |
| **Na Azide** | **0.06%** |
| **P1 Primer** | **40 NM** |

### Additional primer sequences used for TMA amplification

Several P1 (T7 promoter) (SEQ ID NOs: 10-18) and P2 (reverse) (SEQ ID NOs: 1-8) primers (Table 3) were evaluated for 3' HCV amplification in the VIDAS Probe assay as described above. Among these, the 1236 (SEQ ID NO: 11)/1259 (SEQ ID NO: 8) primer pair was optimal, and derivatives of these (e.g., SEQ ID NO: 27 derived from 1236, SEQ ID NO: 37 derived from 1259) were also tested by NASBA (described below). Primer 1279 (SEQ ID NO: 15) also worked well for TMA and NASBA.

**Table 2: Primer sequences**

| |
|---|
| P2 Primers |
| 1206 (SEQ ID NO: 1) GCTCCATCTTAGCCCTAGTC |
| 1242 (SEQ ID NO: 2) TCTTAGCCCTAGTCA |
| 1243 (SEQ ID NO: 3) TTAGCCCTAGTCACG |
| 1244 (SEQ ID NO: 4) AGCCCTAGTCACGGC |
| 1245 (SEQ ID NO: 5) CCCTAGTCACGGCTA |
| 1257 (SEQ ID NO: 6) AGCCCTAGTCACGGCTAG |
| 1258 (SEQ ID NO: 7) AGCCCTAGTCACGGCTAGC |
| 1259 (SEQ ID NO: 8) CCCTAGTCACGGCTAGC |
| P1 primers |
| 1211 (SEQ ID NO: 10) AGGCCAGTATCAGCACTCTC |
| 1236 (SEQ ID NO: 11) AGGCCAGTAACGGCACTCTCTGC |
| 1237 (SEQ ID NO:12) AGGCCAGTATCGGCACTCTCTG |
| 1238 (SEQ ID NO:13) AGGCCAGTAACGGCACTCUCTGC |
| 1278 (SEQ ID NO:14) CAGTATCGGCACTCTCTGCAGT |
| 1279 (SEQ ID NO: 15) CCAGTATCGGCACTCTCTGCAG |
| 1280 (SEQ ID NO: 16) AGGCCAGTATCGGCACTCTCTGC |
| 1281 (SEQ ID NO: 17) AGTATCGGCACTCTCTGCAGT |
| 1282 (SEQ ID NO: 18) CAGTAACGGCACTCTCTGCAGT |

### NASBA amplification of 3' HCV

The 1236/1259 (P1/P2) primer pair, which performed optimally in the TMA assay, was analyzed for use in NASBA amplification. Additional primers providing a larger predicted amplicon were also tested, for example primer P1: 1211 (nt83-64) (SEQ ID NO: 10) and primer P2:VP1206 (nt5-24) (SEQ ID NO: 1).
For detection of amplicons produced by NASBA, probes were designed to bind to a region corresponding to a combination of the VIDAS Probe amplicon capture probe and amplicon detection probe sites from the previous example. Probes were based upon an HCV base sequence of either TGAAAGGTCCGTGAGCCGC (nt 36-54) (SEQ ID NO: 43) or TGTGAAAGGTCCGTGAGCCGC (nt34-54) (SEQ ID NO: 44). These probes were tested as capture probes (EG6, EG7) in the ECL assay (see below) or as the amplicon-binding ("loop") portion of molecular beacons (MB140 (EG18) (SEQ ID NO: 24); MB102 (Q1) (SEQ ID NO: 41)
ECL (electrochemiluminescence) is a NASBA (bioMerieux, Inc., Durham, NC) endpoint detection system that detects amplicons after sequence-specific capture. The ECL system utilizes an ECL detection tag ("ECL tail") linked to the 5' end of a P2 primer. Utilizing an ECL detection tag (SEQ ID NO: 9) with above-described primers and capture probe EG7, amplification was considerable and better with the 1259/1236 pair than 1206/1211 (Table 3).

**Table 3: Performance of various ECL-tagged P1 and P2 primers (normalized fluorescence values)**

| | HCV dilution | | | | | |
|---|---|---|---|---|---|---|
| P2, P1 | 1 | 10x | 100x | 1000x | 10000x | neg |
| 1259ECL,1236 | **2.8E+07** | **3.3E+06** | **3.3E+04** | **9.9E+02** | **1.2E+02** | 1.0E+00 |
| 1206ECL, 1211 | 8.6E+02 | 2.9E+03 | 1.0E+00 | 1.0E+00 | 1.0E+00 | 1.0E+00 |

Similarly, in the absence of ECL tags the 1236 and 1259 primers performed better than 1206 and 1211 in NASBA reactions. As shown in table 4, the 1236/1259 pair performed well.

**Table 4: Performance of various P1 and P2 primers (normalized fluorescence values).**

| | | | | |
|---|---|---|---|---|
| P1 Primer: | 1211 | 1211 | 1236 | 1236 |
| P2 Primer: | 1206 | 1259 | 1206 | 1259 |
| Detection: | -0.0227978 | 0.0323256 | 0.0336202 | **0.7147294** |

ECL detection is presumed to be relatively insensitive to secondary structure, suggesting that the difference in sensitivity is more likely due to increased amplification than increased detection.

### Modified P2 primer designs

In the above assays, it was also found that addition of the 20-nucleotide universal ECL tag to the P2 primer 1259 increased performance. Approximately a 10-fold increase in sensitivity was observed when using the ECL tail on the 1259 P2 primer, as compared to the P2 primer without the ECL tail (as shown in table 5, tagged detection of 10x-diluted sample is roughly equivalent to untagged detection of 1x sample).

**Table 5: Comparison of ECL-tagged vs. untagged P2 primers (P1 used is 1236, probe is EG18, fluorescence values are normalized):**

| | | | | | | |
|---|---|---|---|---|---|---|
| HCV dilution: | 1 | | 10x | | neg | |
| ECL tag | no | yes | no | yes | no | yes |
| Detection | 0.80 | **3.58** | 0.13 | **0.97** | 0.09 | **0.09** |

ECL assays using an HCV-specific probe (i.e., not recognizing the ECL tag) also demonstrated improved performance with the tag, suggesting that this increase in performance was likely due to increased amplification (Table 6).

**Table 6: Comparison of ECL-tagged 1259 vs. untagged, in ECL assay (P1 used is 1236, capture probe is EG7, fluorescence values are normalized):**

| | HCV dilution | | | | |
|---|---|---|---|---|---|
| P2 | 1 | 10x | 100x | 1000x | neg |
| ECL-tagged 1259 | **3.2E+07** | **4.4E+06** | **2.0E+05** | **3.1E+03** | 1 |
| 1259 | 2.1E+06 | 6.9E+05 | 2.1E+04 | 1.3E+01 | 1 |

One likely explanation for the effect of the tag is that the HCV amplicon is very small (70 nt without tag, increased to 90 with tag), and small amplicons may be relatively inefficient in NASBA. Six P2 primers were designed to test whether additional extensions (of 10 or 20 additional nucleotides) to the ECL tag (either homologous to the target or heterologous) could improve amplification. None of the additional heterologous sequences increased performance, and additional homologous sequence decreased performance.

### Additional P1 primers for NASBA

Another set of P1 (promoter) primers was tested for function in NASBA, as listed in Table 7. This set of primers spans, and moves in the 3' direction in one-nucleotide increments, from the position of primer 1236 to (at the 3' end of the final primer P1.89) the position 3 bases from the probe-binding site. Of this set of primers, the best functioning (comparably to primer 1236) to produce detectable signal were P1.63, P1.82, P1.83, P1.84, and P1.85. The P2 used for these tests was EG4 and probe was EG18 (fluorescence values were normalized).

**Table 7**

| |
|---|
| P1.63 (SEQ ID NO: 27) AGACCAGTTACGGCACTCTCTGC |
| P1.81 (SEQ ID NO: 28) GACCAGTTACGGCACTCTCTGCA |
| P1.82 (SEQ ID NO: 29) ACCAGTTACGGCACTCTCTGCAG |
| P1.83 (SEQ ID NO: 30) CCAGTTACGGCACTCTCTGCAGT |
| P1.84 (SEQ ID NO: 31) CAGTTACGGCACTCTCTGCAGTC |
| P1.85 (SEQ ID NO: 32) AGTTACGGCACTCTCTGCAGTCA |
| P1.86 (SEQ ID NO: 33) GTTACGGCACTCTCTGCAGTCAT |
| P1.87 (SEQ ID NO: 34) TTACGGCACTCTCTGCAGTCATG |
| P1.88 (SEQ ID NO: 35) TACGGCACTCTCTGCAGTCATGC |
| P1.89 (SEQ ID NO: 36) ACGGCACTCTCTGCAGTCATGCG |

**Table 8: Amplification with various P1 primers**

| | | | | | | | | | | |
|---|---|---|---|---|---|---|---|---|---|---|
| **Primer** | **P1.63** | **P1.81** | **P1.82** | **P1.83** | **P1.84** | **P1.85** | **P1.86** | **P1.87** | **P1.88** | **P1.89** |
| **Signal:** | 5.250 | 3.184 | 4.886 | 5.099 | 5.239 | 3.746 | 2.668 | 1.995 | 0.025 | 0.142 |

## Claims

1. An isolated nucleic acid **characterised in that** it consists of a nucleotide sequence selected from SEQ ID NO: 11, SEQ ID NO: 27, SEQ ID NO: 29, SEQ ID NO: 30, SEQ ID NO: 31 and SEQ ID NO: 32.

2. An isolated nucleic acid **characterised in that** it comprises:
(i) a promoter oligonucleotide that, when in double-stranded form, can function as a T7 promoter;
and
(ii) a primer nucleic acid corresponding to a portion of the 3' non-translating region (3'-NTR) of Hepatitis C Virus (HCV), having a 5' end and a 3' end and consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
wherein the promoter oligonucleotide is linked at its 3' end to the 5' end of the primer nucleic acid.

3. An isolated nucleic acid as claimed in claim 2 wherein the promoter oligonucleotide has the nucleic acid sequence set forth in SEQ ID NO: 38.

4. An isolated nucleic acid as claimed in claim 2 wherein the promoter oligonucleotide has the nucleic acid sequence set forth in SEQ ID NO: 23.

5. A method of assaying for the presence of HCV in a nucleic acid sample **characterised in that** it comprises:
(a) amplifying a selected HCV nucleic acid to form an HCV amplification product, utilizing as a primer of amplification a nucleic acid consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
and
(b) detecting the presence of amplified product.

6. A method of assaying for the presence of HCV in a nucleic acid sample **characterised in that** it comprises:
(a) amplifying a selected HCV nucleic acid to form an HCV amplification product, utilizing as a primer of amplification a nucleic acid comprising:
(i) a promoter oligonucleotide that, when in double-stranded form, can function as a T7 promoter;
and
(ii) a primer nucleic acid corresponding to a portion of the 3'-NTR of HCV, having a 5' end and a 3' end and consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
wherein the promoter oligonucleotide is linked at its 3' end to the 5' end of the primer nucleic acid;
and
(b) detecting the presence of amplified product.

7. A method as claimed in claim 5 wherein the amplification utilizes a first primer consisting of the nucleotide sequence set forth in SEQ ID NO: 11 and a second primer comprising the nucleotide sequence set forth in SEQ ID NO: 8.

8. A method as claimed in claim 7 wherein the first primer further comprises, at its 5' end, a promoter oligonucleotide that, when in double-stranded form, can function as a T7 promoter.

9. A method as claimed in claim 8 wherein the T7 promoter has the sequence set forth in SEQ ID NO: 38.

10. A method as claimed in claim 5 wherein the amplification utilizes a first primer and a second primer, the said second primer comprising: (a) a tag oligonucleotide having a 5' end and a 3' end, and (b) a primer nucleic acid having a 5' end and a 3' end and comprising a nucleotide sequence selected from SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8, wherein the tag oligonucleotide consists of 10 to 25 randomly selected nucleotides heterologous to HCV and is linked at its 3' end to the 5' end of the primer nucleic acid.

11. A method as claimed in claim 5 wherein the amplification utilizes a first primer and a second primer, the said first primer comprising (a) a promoter oligonucleotide that, when in double-stranded form, can function as a T7 promoter, and (b) a primer nucleic acid corresponding to a portion of the 3'-NTR of HCV, having a 5' end and a 3' end and consisting of a nucleotide sequence selected from SEQ ID NO: 11, SEQ ID NO: 27, SEQ ID NO: 29, SEQ ID NO: 30, SEQ ID NO: 31 and SEQ ID NO: 32, wherein the promoter oligonucleotide is linked at its 3' end to the 5' end of the primer nucleic acid.

12. A method as claimed in claim 5 wherein the detecting is performed by hybridizing to the amplification product a detectable probe.

13. A method as claimed in claim 12 wherein the detectable probe comprises a nucleic acid comprising a nucleotide sequence selected from SEQ ID NO: 21, SEQ ID NO: 22, SEQ ID NO: 24, SEQ ID NO: 41, SEQ ID NO: 43 and SEQ ID NO: 44.

14. A method as claimed in claim 5 wherein the detecting is performed by capturing the amplification product on an immobilised capture probe.

15. A method as claimed in claim 14 wherein the capture probe comprises a nucleic acid comprising a nucleotide sequence set forth in SEQ ID NO: 19.

16. A method as claimed in claim 5 wherein the amplification is performed by isothermal amplification or thermocyclic amplification.

17. A method of detecting the presence of HCV nucleic acid in a sample **characterised in that** it comprises:
hybridizing with the sample, under selective hybridization conditions, a nucleic acid consisting of a nucleotide sequence selected from SEQ ID NO: 11, SEQ ID NO: 27, SEQ ID NO: 29, SEQ ID NO: 30, SEQ ID NO: 31 and SEQ ID NO: 32.

18. A method of detecting the presence of HCV nucleic acid in a sample **characterised in that** it comprises:
hybridizing with the sample, under selective hybridization conditions, a nucleic acid comprising:
(i) a promoter oligonucleotide that, when in double-stranded form, can function as a T7 promoter;
and
(ii) a primer nucleic acid corresponding to a portion of the 3'-NTR of HCV, having a 5' end and a 3' end and consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
wherein the promoter oligonucleotide is linked at its 3' end to the 5' end of the primer nucleic acid.

19. A method of quantifying the amount of HCV nucleic acid in a sample **characterised in that** it comprises:
(a) amplifying selected HCV nucleic acid to form an HCV amplification product, utilizing as a primer of amplification a nucleic acid consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
and
(b) quantifying the amount of amplified product.

20. A method of quantifying the amount of HCV nucleic acid in a sample **characterised in that** it comprises:
(a) amplifying selected HCV nucleic acid to form an HCV amplification product, utilizing as a primer of amplification a nucleic acid comprising:
(i) a promoter oligonucleotide that, when in double-stranded form, can function as a T7 promoter;
and
(ii) a primer nucleic acid corresponding to a portion of the 3'-NTR of HCV, having a 5' end and a 3' end and consisting of a nucleotide sequence selected from SEQ ID NO: 11, SEQ ID NO: 27, SEQ ID NO: 29, SEQ ID NO: 30, SEQ ID NO: 31 and SEQ ID NO: 32;
wherein the promoter oligonucleotide is linked at its 3' end to the 5' end of the primer nucleic acid;
and
(b) quantifying the amount of amplified product.

21. A method as claimed in claim 19 wherein the amplification utilizes a first primer consisting of the nucleotide sequence set forth in SEQ ID NO: 11 and a second primer comprising the nucleotide sequence set forth in SEQ ID NO: 8.

## Patentansprüche

1. Isolierte Nukleinsäure, **dadurch gekennzeichnet, dass** sie besteht aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32.

2. Isolierte Nukleinsäure, **dadurch gekennzeichnet, dass** sie umfasst:
(i) Ein Promotor-Oligonukleotid, welches, wenn es in doppelsträngiger Form vorliegt, als ein T7-Promotor wirken kann;
und
(ii) eine Primer-Nukleinsäure, die einem Teil des 3' nicht translatierten Abschnitts (3'-NTR) des Hepatitis C-Virus (HCV) entspricht, mit einem 5'-Ende und einem 3'-Ende, und bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32;
wobei das Promotor-Oligonukleotid an seinem 3'-Ende an das 5'-Ende der Primer-Nukleinsäure verknüpft ist.

3. Isolierte Nukleinsäure, wie sie in Anspruch 2 beansprucht wird, wobei das Promotor-Oligonukleotid die Nukleinsäure nach SEQ ID NO: 38 aufweist.

4. Isolierte Nukleinsäure, wie sie in Anspruch 2 beansprucht wird, wobei das Promotor-Oligonukleotid die Nukleinsäure nach SEQ ID NO: 23 aufweist.

5. Verfahren zum Nachweis des Vorliegens von HCV in einer Nukleinsäureprobe, **dadurch gekennzeichnet, dass** es umfasst:
(a) Amplifizieren einer ausgewählten HCV-Nukleinsäure, um ein HCV-Amplifikationsprodukt zu bilden, Verwenden einer Nukleinsäure, bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32 als Primer für die Amplifikation der Nukleinsäure;
und
(b) Nachweisen des Vorliegens des amplifizierten Produktes.

6. Verfahren zum Nachweis des Vorliegens von HCV in einer Nukleinsäureprobe, **dadurch gekennzeichnet, dass** es umfasst:
(a) Amplifizieren einer selektierten HCV-Nukleinsäure, um ein HCV-Amplifikationsprodukt zu bilden, welches als Primer zur Amplifikation einer Nukleinsäure eingesetzt wird, umfassend:
(i) Ein Promotor-Oligonukleotid, welches, wenn es doppelsträngiger Form vorliegt, als T7-Promotor wirken kann;
und
(ii) Eine Primer-Nukleinsäure, welche einem Teil des 3'-NTR von HCV entspricht, mit einem 5'-Ende und einem 3'-Ende und bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32;
wobei das Promotor-Oligonukleotid an seinem 3'-Ende an das 5'-Ende der Primer-Nukleinsäure verknüpft ist;
und
(b) Nachweisen des Vorliegens des amplifizierten Produktes.

7. Verfahren, wie es im Anspruch 5 beansprucht wird, wobei die Amplifikation einen ersten Primer verwendet, der aus der Nukleotidsequenz nach SEQ ID NO: 11 besteht und einen zweiten Primer, der die Nukleotidsequenz nach SEQ ID NO: 8 umfasst.

8. Verfahren, wie es in Anspruch 7 beansprucht wird, wobei der erste Primer weiterhin, an einem 5'-Ende, ein Promotor-Oligonukleotid aufweist, welches, wenn es in doppelsträngiger Form vorliegt, als ein T7-Promotor wirken kann.

9. Verfahren, wie es im Anspruch 8 beansprucht wird, wobei der T7-Promotor die Sequenz nach SEQ ID NO: 38 besitzt.

10. Verfahren, wie es im Anspruch 5 beansprucht wird, wobei die Amplifikation einen ersten Primer und einen zweiten Primer einsetzt, wobei der zweite Primer aufweist: (a) ein Tag-Oligonukleotid mit einem 5'-Ende und einem 3'-Ende, und (b) eine Primer-Nukleinsäure mit einem 5'-Ende und einem 3'-Ende, und eine Nukleotidsequenz umfasst, ausgewählt aus SEQ ID NO: 11, SEQ ID NO: 27, SEQ ID NO: 29, SEQ ID NO: 30, SEQ ID NO: 31 und SEQ ID NO: 32, wobei das Tag-Oligonukleotid aus 10 bis 25 zufällig ausgewählten Nukleotiden besteht, die zum HCV heterolog sind und es an seinem 3'-Ende an das 5'-Ende der Primer-Nukleinsäure verknüpft ist.

11. Verfahren, wie es im Anspruch 5 beansprucht wird, wobei die Amplifikation einen ersten Primer und einen zweiten Primer verwendet, wobei der erste Primer umfasst: (a) ein Promotor-Oligonukleotid, welches, wenn es in doppelsträngiger Form vorliegt, als ein T7-Promotor wirken kann und (b) eine Primer-Nukleinsäure, die einem Teil der 3'-NTR von HCV entspricht, mit einem 5'-Ende und einem 3'-Ende, und bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32, wobei das Promotor-Oligonukleotid an seinem 3'-Ende an das 5'-Ende der Primer-Nukleinsäure verknüpft ist.

12. Verfahren, wie es im Anspruch 5 beansprucht wird, wobei der Nachweis durch Hybridisieren einer nachweisbaren Sonde an das Amplifikationsprodukt durchgeführt wird.

13. Verfahren, wie es im Anspruch 12 beansprucht wird, wobei die nachweisbare Sonde eine Nukleinsäure umfasst, die eine Nukleotidsequenz aufweist, ausgewählt aus SEQ ID NO: 21, SEQ ID NO: 22, SEQ ID NO: 24, SEQ ID NO: 41, SEQ ID NO: 43 und SEQ ID NO: 44.

14. Verfahren, wie es im Anspruch 5 beansprucht wird, wobei der Nachweis durch Fangen des Amplifikationsproduktes auf einer immobilisierten Fängersonde durchgeführt wird.

15. Verfahren, wie es im Anspruch 14 beansprucht wird, wobei die Fängersonde eine Nukleinsäure aufweist, die eine Nukleotidsequenz nach SEQ ID NO: 19 aufweist.

16. Verfahren, wie es im Anspruch 5 beansprucht wird, wobei die Amplifikation durch isothermale Amplifikation oder thermozyklische Amplifikation durchgeführt wird.

17. Verfahren zum Nachweis des Vorliegens der HCV-Nukleinsäure in einer Probe, **dadurch gekennzeichnet, dass** es umfasst:
Hybridisieren mit der Probe, unter selektiven Hybridisierungsbedingungen, einer Nukleinsäure, bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32.

18. Verfahren zum Nachweis des Vorliegens einer HCV-Nukleinsäure in einer Probe, **dadurch gekennzeichnet, dass** es umfasst:
Hybridisieren mit der Probe, unter selektiven Hybridisierungsbedingungen, einer Nukleinsäure, umfassend:
(i) Ein Promotor-Oligonukleotid, welches, wenn es in doppelsträngiger Form vorliegt, als ein T7-Promotor wirken kann;
und
(ii) eine Primer-Nukleinsäure, die einem Abschnitt des 3'-NTR von HCV entspricht, mit einem 5'-Ende und einem 3'-Ende, und bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32;
wobei das Promotor-Oligonukleotid an seinem 3'-Ende an das 5'-Ende der Primer-Nukleinsäure verknüpft wird.

19. Verfahren zum Quantifizieren der Menge der HCV-Nukleinsäure in einer Probe, **dadurch gekennzeichnet, dass** es umfasst:
(a) Amplifizieren der ausgewählten HCV-Nukleinsäure, um ein HCV-Amplifikationsprodukt zu bilden,
Verwenden, als einen Primer zur Amplifikation, einer Nukleinsäure, bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32;
und
(b) Quantifizieren der Menge des amplifizierten Produktes.

20. Verfahren zum Quantifizieren der Menge einer HCV-Nukleinsäure in einer Probe, **dadurch gekennzeichnet, dass** es umfasst:
(a) Amplifizieren der ausgewählten HCV-Nukleinsäure, um ein HCV-Amplifizierungsprodukt zu bilden,
Verwenden, als einen Primer zur Amplifizierung, einer Nukleinsäure, umfassend:
(i) Ein Promotor-Oligonukleotid, welches, wenn es in doppelsträngiger Form vorliegt, als ein T7-Promotor wirken kann;
und
(ii) eine Primer Nukleinsäure, die einem Abschnitt der 3'-NTR von HCV entspricht, mit einem 5'-Ende und einem 3'-Ende, und bestehend aus einer Nukleotidsequenz, ausgewählt aus SEQ ID NO: 11, SEQ ID NO: 27, SEQ ID NO: 29, SEQ ID NO: 30, SEQ ID NO: 31 und SEQ ID NO: 32;
wobei das Promotor-Oligonukleotid an seinem 3'-Ende an das 5'-Ende der Primer-Nukleinsäure verknüpft wird;
und
(b) Quantifizieren der Menge des amplifizierten Produktes.

21. Verfahren, wie es im Anspruch 19 beansprucht wird, wobei die Amplifikation einen ersten Primer verwendet, bestehend aus der Nukleotinsequenz nach SEQ ID NO: 11, und einem zweiten Primer, umfassend die Nukleotidsequenz nach SEQ ID NO: 8.

## Revendications

1. Acide nucléique isolé, **caractérisé en ce qu'**il consiste en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32.

2. Acide nucléique isolé, **caractérisé en ce qu'**il comprend :
(i) un oligonucléotide promoteur qui, lorsqu'il est sous une forme double brin, peut fonctionner comme un promoteur T7 ;
et
(ii) un acide nucléique amorce correspondant à une partie de la région non traduite 3' (3'-NTR) du virus de l'hépatite C (HCV), ayant une extrémité 5' et une extrémité 3', et consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
où l'oligonucléotide promoteur est lié au niveau de son extrémité 3' à l'extrémité 5' de l'acide nucléique amorce.

3. Acide nucléique isolé selon la revendication 2, dans lequel l'oligonucléotide promoteur possède la séquence d'acide nucléique décrite dans SEQ ID NO: 38.

4. Acide nucléique isolé selon la revendication 2, dans lequel l'oligonucléotide promoteur possède la séquence d'acide nucléique décrite dans SEQ ID NO: 23.

5. Procédé pour analyser la présence du HCV dans un échantillon d'acide nucléique, **caractérisé en ce qu'**il consiste à :
(a) amplifier un acide nucléique du HCV sélectionné afin de former un produit d'amplification du HCV, en utilisant en tant qu'amorce d'amplification un acide nucléique consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
et
(b) détecter la présence du produit amplifié.

6. Procédé pour analyser la présence du HCV dans un échantillon d'acide nucléique, **caractérisé en ce qu'**il consiste à :
(a) amplifier un acide nucléique du HCV sélectionné afin de former un produit d'amplification du HCV, en utilisant en tant qu'amorce d'amplification un acide nucléique comprenant :
(i) un oligonucléotide promoteur qui, lorsqu'il est sous une forme double brin, peut fonctionner comme un promoteur T7 ;
et
(ii) un acide nucléique amorce correspondant à une partie de la 3'-NTR du HCV, ayant une extrémité 5' et une extrémité 3', et consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
où l'oligonucléotide promoteur est lié au niveau de son extrémité 3' à l'extrémité 5' de l'acide nucléique amorce ;
et
(b) détecter la présence du produit amplifié.

7. Procédé selon la revendication 5, dans lequel l'amplification utilise une première amorce consistant en la séquence de nucléotides décrite dans SEQ ID NO: 11 et une seconde amorce comprenant la séquence de nucléotides décrite dans SEQ ID NO: 8.

8. Procédé selon la revendication 7, dans lequel la première amorce comprend en outre, à son extrémité 5', un oligonucléotide promoteur qui, lorsqu'il est sous une forme double brin, peut fonctionner comme un promoteur T7.

9. Procédé selon la revendication 8, dans lequel le promoteur T7 possède la séquence décrite dans SEQ ID NO: 38.

10. Procédé selon la revendication 5, dans lequel l'amplification utilise une première amorce et une seconde amorce, ladite seconde amorce comprenant : (a) un oligonucléotide marqueur ayant une extrémité 5' et une extrémité 3', et (b) un acide nucléique amorce ayant une extrémité 5' et une extrémité 3' et comprenant une séquence de nucléotides sélectionnée parmi SEQ ID NO: 1, SEQ ID NO: 2, SEQ ID NO: 3, SEQ ID NO: 4, SEQ ID NO: 5, SEQ ID NO: 6 , SEQ ID NO: 7 et SEQ ID NO: 8, où l'oligonucléotide marqueur consiste en 10 à 25 nucléotides sélectionnés de manière aléatoire, hétérologues pour le HCV, et est lié au niveau de son extrémité 3' à l'extrémité 5' de l'acide nucléique amorce.

11. Procédé selon la revendication 5, dans lequel l'amplification utilise une première amorce et une seconde amorce, ladite première amorce comprenant (a) un oligonucléotide promoteur qui, lorsqu'il est sous une forme double brin, peut fonctionner comme un promoteur T7, et (b) un acide nucléique amorce correspondant à une partie de la 3'-NTR du HCV, ayant une extrémité 5' et une extrémité 3', et consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32, où l'oligonucléotide promoteur est lié au niveau de son extrémité 3' à l'extrémité 5' de l'acide nucléique amorce.

12. Procédé selon la revendication 5, dans lequel la détection est réalisée en hybridant une sonde détectable au produit d'amplification.

13. Procédé selon la revendication 12, dans lequel la sonde détectable comprend un acide nucléique comprenant une séquence de nucléotides sélectionnée parmi SEQ ID NO: 21, SEQ ID NO: 22, SEQ ID NO: 24, SEQ ID NO: 41, SEQ ID NO: 43 et SEQ ID NO: 44.

14. Procédé selon la revendication 5, dans lequel la détection est réalisée en capturant le produit d'amplification sur une sonde de capture immobilisée.

15. Procédé selon la revendication 14, dans lequel la sonde de capture comprend un acide nucléique comprenant une séquence de nucléotides décrite dans SEQ ID NO: 19.

16. Procédé selon la revendication 5, dans lequel l'amplification est réalisée par une amplification isotherme ou une amplification thermocyclique.

17. Procédé pour détecter la présence d'un acide nucléique du HCV dans un échantillon, **caractérisé en ce qu'**il consiste à :
hybrider à l'échantillon, dans des conditions d'hybridation sélectives, un acide nucléique consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32.

18. Procédé pour détecter la présence d'un acide nucléique du HCV dans un échantillon, **caractérisé en ce qu'**il consiste à :
hybrider à l'échantillon, dans des conditions d'hybridation sélectives, un acide nucléique comprenant :
(i) un oligonucléotide promoteur qui, lorsqu'il est sous une forme double brin, peut fonctionner comme un promoteur T7 ; et
(ii) un acide nucléique amorce correspondant à une partie de la 3'-NTR du HCV, ayant une extrémité 5' et une extrémité 3', et consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
où l'oligonucléotide promoteur est lié au niveau de son extrémité 3' à l'extrémité 5' de l'acide nucléique amorce.

19. Procédé pour quantifier la quantité d'acide nucléique du HCV dans un échantillon, **caractérisé en ce qu'**il consiste à :
(a) amplifier un acide nucléique du HCV sélectionné afin de former un produit d'amplification du HCV, en utilisant en tant qu'amorce d'amplification un acide nucléique consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
et
(b) quantifier la quantité de produit amplifié.

20. Procédé pour quantifier la quantité d'acide nucléique du HCV dans un échantillon, **caractérisé en ce qu'**il consiste à :
(a) amplifier un acide nucléique du HCV sélectionné afin de former un produit d'amplification du HCV, en utilisant en tant qu'amorce d'amplification un acide nucléique comprenant :
(i) un oligonucléotide promoteur qui, lorsqu'il est sous une forme double brin, peut fonctionner comme un promoteur T7 ;
et
(ii) un acide nucléique amorce correspondant à une partie la 3'-NTR du HCV, ayant une extrémité 5' et une extrémité 3', et consistant en une séquence de nucléotides sélectionnée parmi SEQ ID NO: 11, SEQ ID NO: 27, SEQ ID NO: 29, SEQ ID NO: 30, SEQ ID NO: 31 et SEQ ID NO: 32 ;
où l'oligonucléotide promoteur est lié au niveau de son extrémité 3' à l'extrémité 5' de l'acide nucléique amorce ;
et
(b) quantifier la quantité de produit amplifié.

21. Procédé selon la revendication 19, dans lequel l'amplification utilise une première amorce consistant en la séquence de nucléotides décrite dans SEQ ID NO: 11 et une seconde amorce comprenant la séquence de nucléotides décrite dans SEQ ID NO: 8.


### SEQUENCE LISTING

<110> Hwa-Tang Thomas Wang; Brian K. Washburn
<120> PRIMER AND PROBE DESIGN FOR EFFICIENT AMPLIFICATION AND DETECTION OF HCV 3' NON-TRANSLATING REGION
<130> 01063
<150> 60/538,814, 60/538,815 60/538,816
<151> 2004-01-23
<160> 43
<210> 1
   <212> Type: DNA
   <213> Organism: Hepatitis C virus
<223> Other information: "1206"
   <400> GCTCCATCTTAGCCCTAGTC
<210> 2
   <212> Type: DNA
   <213> Organism: Hepatitis C virus
<223> Other information: "1242"
   <400> TCTTAGCCCTAGTCA
<210> 3
   <212> Type: DNA
   <213> Organism: Hepatitis C virus
<223> Other information: "1243"
<400> TTAGCCCTAGTCACG
<210> 4
   <212> Type: DNA
   <213> Organism: Hepatitis C virus
<223> Other information: "1244"
<400> AGCCCTAGTCACGGC
<210> 5
   <212> Type: DNA
   <213> Organism: Hepatitis C virus
<223> Other information: "1245"
<400> CCCTAGTCACGGCTA
<210> 6
   <212> Type: DNA.
   <213> Organism: Hepatitis C virus
<223> Other information: "1257"
<400> AGCCCTAGTCACGGCTAG
<210> 7
   <212> Type: DNA
   <213> Organism: Hepatitis C virus
<223> Other information: "1258"
<400> AGCCCTAGTCACGGCTAGC
<210> 8
   <212> Type: DNA
   <213> Organism: Hepatitis C virus
<223> Other information: "1259"
<400> CCCTAGTCACGGCTAGC
<210> 9
   <212> Type: DNA
   <213> Organism: artificial
<223> Other information: "ECL tag"
<400> GATGCAAGGTCGCATATGAG
<210> 10
   <212> Type: DNA
   <213> Organism: Hepatitis C virus
<223> Other information: "1211"
<400> AGGCCAGTATCAGCACTCTC
<210> 11
   <212> Type: DNA
   <213> Organism: Hepatitis C virus
<223> Other information: "1236"
<400> AGGCCAGTAACGGCACTCTCTGC
<210> 12
   <212> Type: DNA
   <213> Organism: Hepatitis C virus
<223> Other information: "1237"
<400> AGGCCAGTATCGGCACTCTCTG
<210> 13
   <212> Type: DNA
   <213> Organism: Hepatitis C virus
<223> Other information: "1238"
<400> AGGCCAGTAACGGCACTCUCTGC
<210> 14
   <212> Type: DNA
   <213> Organism: Hepatitis C virus
<223> Other information: "1278"
<400> CAGTATCGGCACTCTCTGCAGT
<210> 15
   <212> Type: DNA
   <213> Organism: Hepatitis C virus
<223> Other information: "1279"
<400> CCAGTATCGGCACTCTCTGCAG
<210> 16
   <212> Type: DNA
   <213> Organism: Hepatitis C virus
<223> Other information: "1280"
<400> AGGCCAGTATCGGCACTCTCTGC
<210> 17
   <212> Type: DNA
   <213> Organism: Hepatitis C virus
<223> Other information: "1281"
<400> AGTATCGGCACTCTCTGCAGT
<210> 18
   <212> Type: DNA
   <213> Organism: Hepatitis C virus
<223> Other information: "1282"
<400> CAGTAACGGCACTCTCTGCAGT
<210> 19
   <212> Type: DNA
   <213> Organism: Hepatitis C virus
<223> Other information: "capB"
<400> GGGCTAAGATGGAGCCACC
<210> 20
   <212> Type: DNA
   <213> Organism: Hepatitis C virus
<223> Other information: "1289"
<400> ACATGATCTGCAGAGA
<210> 21
   <212> Type: DNA
   <213> Organism: Hepatitis C virus
<223> Other information: : "1246"
<400> CTGTGAAAGGTCCG
<210> 22
<212> Type: DNA
   <213> Organism: Hepatitis C virus
<223> Other information: "1247"
<400> TGAGCCGCATGACTGC
<210> 23
   <212> Type: DNA
   <213> Organism: T7
<223> Other information: "T7 promoter sequence"
<400> AATTTAATACGACTCACTATAGGG
<210> 24
   <212> Type: DNA
   <213> Organism: Hepatitis C virus
<223> Other information: "MB 140"
<400> CGATGCTGAAAGGTCCGTGAGCCGCGCATCG
<210> 25
   <212> Type: DNA
   <213> Organism: Hepatitis C virus Type I
<223> Other information: 3' NTR
<400>

GGUGGCUCCAUCUUAGCCCUAGUCACGGCU
AGCUGUGAAAGGUCCGUGAGCCGCAUGACU
GCAGAGAGUGCUGAUACUGGCCUCUCUGCA
GAUCAUGU

<210> 26
   <212> Type: DNA
   <213> Organism: Hepatitis C virus Type II
<223> Other information: 3'NTR; underlining indicates difference from Type I
<400> 
GGUGGCUCCAUCUUAGCCCUAGUCACGGCU
AGCUGUGAAGGUCCGUGAGCCGCAUGACU
GCAGAGAGUGCCGUAACUGGUCUCUCUGCA
GAUCAUGU
<210> 27
   <212> Type: DNA
   <213> Organism: Hepatitis C virus
<223> Other information: "P1.63"
<400> AGACCAGTTACGGCACTCTCTGC
<210> 28
   <212> Type: DNA
   <213> Organism: Hepatitis C virus
<223> Other information: "P1.81"
<400> GACCAGTTACGGCACTCTCTGCA
<210> 29
<212> Type: DNA
   <213> Organism: Hepatitis C virus
<223> Other information: "P1.82"
<400> ACCAGTTACGGCACTCTCTGCAG
<210> 30
   <212> Type: DNA
   <213> Organism: Hepatitis C virus
<223> Other information: "P1.83"
<400> CCAGTTACGGCACTCTCTGCAGT
<210> 31
<212> Type: DNA
   <213> Organism: Hepatitis C virus
<223> Other information: "P1.84"
<400> CAGTTACGGCACTCTCTGCAGTC
<210> 32
   <212> Type: DNA
   <213> Organism: Hepatitis C virus
<223> Other information: "P1.85"
<400> AGTTACGGCACTCTCTGCAGTCA
<210> 33
   <212> Type: DNA
   <213> Organism: Hepatitis C virus
<223> Other information: "P1.86"
<400> GTTACGGCACTCTCTGCAGTCAT
<210> 34
   <212> Type: DNA
   <213> Organism: Hepatitis C virus
<223> Other information: "P1.87"
<400> TTACGGCACTCTCTGCAGTCATG
<210> 35
   <212> Type: DNA
   <213> Organism: Hepatitis C virus
<223> Other information: "P1.88"
<400> TACGGCACTCTCTGCAGTCATGC
<210> 36
   <212> Type: DNA
   <213> Organism: Hepatitis C virus
<223> Other information: "P1.89"
<400> ACGGCACTCTCTGCAGTCATGCG
<210> 37
   <212> Type: DNA
   <213> Organism: artificial
<223> Other information: "ECL+1259"
<400> GATGCAAGGTCGCATATGAGCCCTAGTCACGGCTAGC
<210> 38
   <212> Type: DNA
   <213> Organism: T7
<223> Other information: "T7 promoter sequence"
<400> AATTCTAATACGACTCACTATAGGG
<210> 39
   <212> Type: DNA
   <213> Organism: Hepatitis C virus
<223> Other information: "MB 110 (Q2)"
<400> GCACTCGTGTGAAAGGTCCGTGAGCCGCCGAGTGC
<210> 40
   <212> Type: DNA
   <213> Organism: Hepatitis C virus
<223> Other information: "MB 114"
<400> CGACTCGTGTGAAAGGTCCGTGAGCCGCCGAGTCG
<210> 41
   <212> Type: DNA
   <213> Organism: artificial
<223> Other information: "MB102 (Q1)"
<400> GCATGCTGTGAAAGGTCCGTGAGCCGCGCATGC
<210> 42
   <212> Type: DNA
   <213> Organism: Hepatitis C virus
<223> Other information: "EG6"
<400> TGTGAAAGGTCCGTGAGCCGC
<210> 43
   <212> Type: DNA
   <213> Organism: Hepatitis C virus
<223> Other information: "EG7"
<400> TGAAAGGTCCGTGAGCCGC